Europäisches Patentamt

(19) European Patent Office

Office européen des brevets

(11) Publication number: **0 156 974**
**B1**

(12) **EUROPEAN PATENT SPECIFICATION**

(45) Date of publication of patent specification: **30.08.89**

(21) Application number: **84114913.1**

(22) Date of filing: **07.12.84**

(51) Int. Cl.⁴: **A 61 M 1/00**

(54) **Three-stage valve for the flow control of body fluids.**

(30) Priority: **08.12.83 US 559392**

(43) Date of publication of application:
**09.10.85 Bulletin 85/41**

(45) Publication of the grant of the patent:
**30.08.89 Bulletin 89/35**

(84) Designated Contracting States:
**DE FR GB IT NL**

(56) References cited:
**EP-A-0 117 050**
**DE-A-2 444 452**
**US-A-3 270 771**
**US-A-3 768 508**
**US-A-3 769 982**

(73) Proprietor: **Cordis Corporation**
**10555 West Flagler Street**
**Miami Florida 33102-5700 (US)**
(73) Proprietor: **Sainte-Rose, Christian**
**14, Rue Corbon**
**F-75015 Paris (FR)**

(72) Inventor: **Hooven, Michael D.**
**14826 S.W. 140th Place**
**Miami Florida 33186 (US)**
Inventor: **Sainte-Rose, Christian**
**14, rue Corbon**
**F-75015 Paris (FR)**

(74) Representative: **Kuhnen, Wacker & Partner**
**Schneggstrasse 3-5 Postfach 1553**
**D-8050 Freising (DE)**

Courier Press, Leamington Spa, England.

## Description

Background of the Invention

The present invention relates to an intracranial pressure relief valve and, more particularly, to a three stage valve which provides either constant pressure or constant flow characteristics in accordance with a fluid pressure differential applied across the value.

Hydrocephalus is a condition in which the body, for any one of a variety of reasons, is unable to relieve itself of excess cerebrospinal fluid (CSF) collected in the ventricles of the brain. The excessive collection of CSF in the ventricular spaces results in an increase in both epidural and intradural pressures. This in turn causes a number of adverse physiological effects including compression of brain tissue, impairment of blood flow in the brain tissue and impairment of the brain's normal metabolism.

Treatment of a hydrocephalic condition frequently involves relieving the abnormally high intracranial pressure. Accordingly, a variety of CSF pressure regulator valves and methods of controlling CSF pressure have been developed which include various forms of check valves, servo valves or combinations thereof. Generally, such valves serve to divert CSF from the ventricles of the brain through a discharge line to some suitable drainage area of the body such as the venous system or the peritoneal cavity. Check valves operate by opening when the difference between CSF pressure and pressure in the discharge line exceeds a predetermined value.

One drawback to the use of a simple check valve in the treatment of hydrocephalus is that such a valve might open in response to normal variations in differential pressure between CSF ventricular pressure and pressure in the discharge line, resulting in hyperdrainage of the ventricular spaces. For example, when a patient stands after lying in a recumbent position, the differential pressure will normally increase by reason of the resulting increased vertical height of the fluid column existing between the head and the selected drainage location. Though such an increase in differential pressure is quite normal, a check valve might respond by opening, thereby allowing undesired hyperdrainage of the ventricular spaces, which, in turn, may result in a potentially serious brain hematoma. Accordingly, it is desirable to provide a hydrocephalus pressure relief valve which is effective in shunting CSF in response to abnormal intracranial pressures while avoiding hyperdrainage in the event of normal variations in body fluid pressures.

From US-A-3 769 982 a physiological drainage system for draining liquids from a source of the human body to a region where it is deposed of is known. The fluid is normally subject to pressure variations within a predetermined nominal range, and abnormally subject to pressure variations in excess thereof extending to a predetermined maximum allowable level. This known system in theform of a valve comprises a housing with a first and a second interior chamber, inlet port means for establishing fluid communication between said first chamber and the source location, outlet port means for establishing fluid communication between said second chamber and the drain location and valving means for regulating fluid flow between said first chamber and the second chamber. Said valving means provide a first condition in which fluid communication between said first and second chambers is prevented and a second condition in which fluid flow between said first and second chambers is established. Further the drainage system of US-A-3 769 982 comprises partition means in said housing including a movable member in form of a diaphragm separating said first and second chambers and being movable in response to the pressure differential therebetween. The movable member is operatively associated with said valving means such that motion of said movable member in response to in increasing pressure differential between fluids in said first and second chambers conditions said valving means from said first condition into said second condition.

The valve known from US-A-3 769 982 should enable the region to be drained at any desired outlet pressure, but to prevent it from being drained excessively because of excessive suction forces. Accordingly the valve known from this prior art provides two positions, a closed and an open position which allows a constant amount of fluid to be drained. When the pressure differential is relatively small, the valve allows the passage of fluid at a flow rate sufficient to maintain a desired ventricular CSF pressure. However, a sudden increase in differential pressure, as would occur when the patient stands, causes the valve to operate essentially as a constant flow device in which passage of fluid is maintained at a relatively constant desired flow rate. A very high differential pressure, as might occur as a result of undesired clogging of the valve, causes the valve to once again operate in a constant pressure mode this time at a higher pressure, thereby preventing CSF pressure from building above a predetermined maximum safe value.

It is, therefore, an object of the present invention to provide a pressure regulator valve which avoids undesired hyperdrainage of the ventricular spaces as a result of normal increases in differential pressure, enabling regulation of the fluid flow rate depending on the differential pressure.

According to the present invention said valving means in said second condition regulates the fluid flow between said first and said second chambers to maintain a first substantially constant predetermined pressure in said first chamber. Further, said valving means provide a third condition in which fluid flow between said first and second chambers is maintained at a substantially constant flow rate and a fourth condition in which fluid flow between said first and second chambers is regulated to maintain a second substantially constant predetermined pressure in said first chamber, wherein said movable member sequentially moves from said first condition through said second and third conditions to said fourth condition whereby, in response to an increasing pressure differential between fluid at the source location and fluid at the drain location, said valving means in said first condition prevents the passage of fluid between said fourth location and the drain location when the source pressure falls below the nominal range, said valving means in said second condition maintains a constant fluid pressure differential between the source location and the drain location when the source pressure is in the nominal range, said valving means in said third condition maintains a desired constant rate of fluid flow between the source location and the drain location when the source pressure is greater than the nominal range but less than the maximum allowable level, and said valving means in said fourth condition maintains a maximum allowable pressure level at the source location. Accordingly, the valve mechanism will be sequentially conditioned to one of the four conditions in response to an increasing pressure differential between fluid at the source location of the body and fluid at the drain location of the body.

The features of the present invention are set forth with particularity in the appended claims. The invention, together with the further objects and advantages thereof, may best be understood by reference to the following description taken in conjunction with the accompanying drawings, in the several figures of which like reference numerals identify like elements, and in which:

Fig. 1 is a perspective view, partially in section, of a hydrocephalus system employing a three stage pressure regulator valve constructed in accordance with the invention, showing such a system implanted within a patient.

Fig. 2 is a plan view, partially in section, of a hydrocephalus system showing the principal elements thereof.

Fig. 3 is a cross-sectional view taken along line 3—3 of figure 2.

Fig. 4 is an exploded perspective view of a three stage pressure regulator valve constructed in accordance with the invention showing the principal elements thereof.

Fig. 5 is a plan view, partially in section, of the three stage pressure regulator valve shown in fig. 4.

Fig. 6 is an enlarged cross-sectional view of the valve shown in fig. 5 taken along line 6-6 thereof.

Fig. 7 is a cross-sectional view, similar to fig. 6, showing the valve in a first constant pressure mode.

Fig. 8 is a cross-sectional view, similar to fig. 6, showing the valve in a constant flow-rate mode.

Fig. 9 is a cross-sectional view, similar to fig. 6, showing the valve in a second constant pressure mode.

Fig. 10 is a graphical representation of certain pressure and flow characteristics of the three stage valve constructed in accordance with the invention useful in understanding the operation thereof.

Description of the Preferred Embodiment

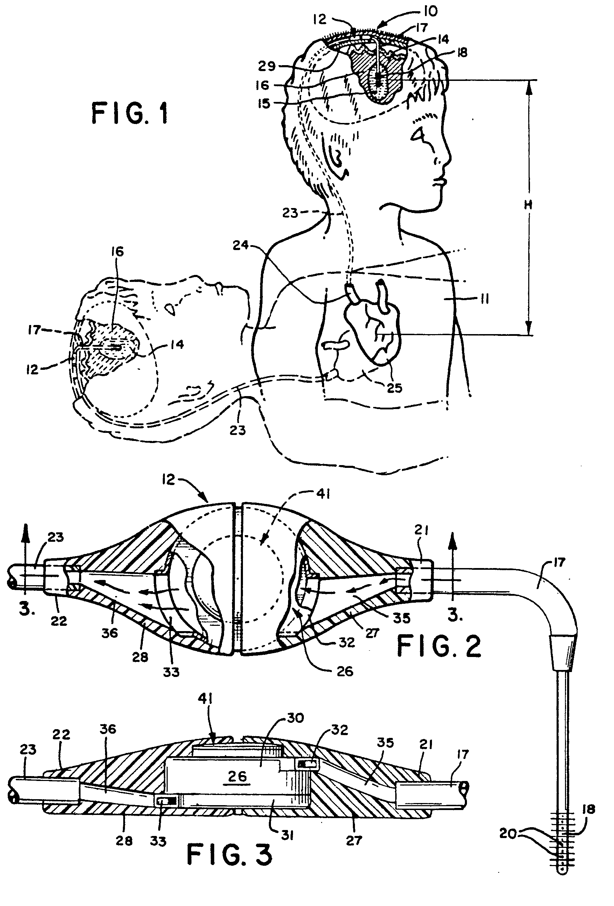

Referring to the drawings, and particularly to fig. 1, a hydrocephalus system 10 for maintaining a desired predetermined intracranial pressure in a patient 11 is illustrated. The system shown includes a three stage pressure relief valve 12 constructed in accordance with the present invention for maintaining the desired intracranial pressure.

Cerebrospinal fluid (CSF) 14 is drained from a ventricle 15 of the brain 16 by means of a ventricular catheter 17. Preferably, the catheter is radio-opaque in order to facilitate its accurate placement within the brain. The distal end 18 of the catheter is provided with a plurality of apertures 20 allowing the passage of CSF therethrough and is positioned in a suitable brain ventricle as illustrated. The other end of the catheter is coupled to the inlet port 21 of the valve thereby establishing fluid communication between the valve and the ventricle. The outlet port 22 of the valve is attached to one end of a drain catheter 23, the opposite end of which discharges into an appropriate location of the patient's body. As illustrated, the drain catheter is threaded through an appropriate vein 24 to terminate within the right atrium of the heart 25. Another drainage location, for example, the peritoneal cavity, could also be selected. When open, the valve allows passage of CSF from the brain ventricles to the selected discharge location thereby relieving CSF pressure caused by excessive accumulation of CSF.

Normally, a pressure differential exists between CSF within the ventricle and fluid in the selected drainage location. Accordingly, a differential pressure will exist between fluids applied to the inlet and outlet ports of the valve. The valve illustrated is of the type which opens when the pressure differential exceeds a predetermined threshold value. Typically, the valve 12 includes means for adjusting the differential pressure threshold at which it opens so that the hydrocephalus system may be adjusted to suit the specific requirements of an individual patient.

While an increased differential pressure may result from the excessive accumulation of CSF in the brain ventricles, such an increase may be a perfectly normal response to ordinary physical activity of the patient. For example, when a patient stands after lying for some time in a recumbent position as illustrated in phantom in fig. 1, the differential pressure will suddenly increase by reason of the sudden increase in vertical height H of the fluid column existing between the distal end of the ventricular catheter 17 and the drainage location. If the relief valve were to open and permit unrestrained fluid flow during this period, hyperdrainage of the ventricle, and a brain hematoma, are possible results. Accordingly, the valve includes means for preventing such unrestricted fluid flow to the drainage location in the event of a sudden increase in the differential pressure.

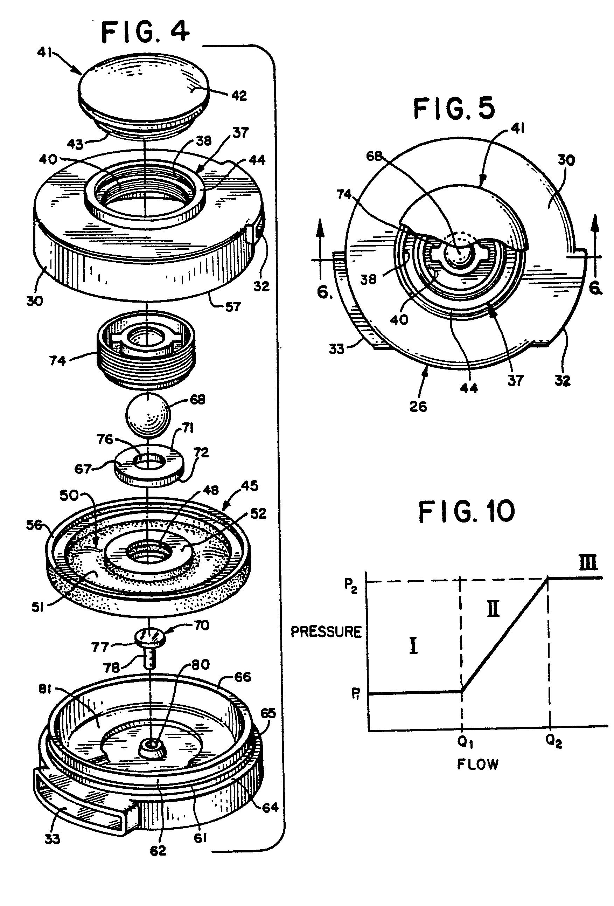

The internal construction and operation of the three stage valve may best be understood by reference to figures 2-6. As illustrated, the valve includes a disc-shaped inner housing 26 fashioned from a durable, biologically compatible material, such as thermoplastic polymers of polyethersulfone or polycarbonates. The inner housing 26 is received within an outer housing comprising two members 27 and 28 formed of silicone rubber or similar material bonded together over the inner housing. The dimensions of the inner and outer housings are selected so as to be compatible with subcutaneous implantation of the valve over the cranium 29.

As is best illustrated in figures 3 and 4, the inner housing includes two circular cup-shaped members 30 and 31, each including a single port 32 and 33 respectively, by means of which fluid can pass to or from the interior region of the housing. In this regard, outer housing members 27 and 28 are provided with internal conduits 35 and 36 respectively, which allow fluid communication between the inlet and outlet ports and the housing 26.

Upper housing member 30 is provided with an aperture 37 through the upper surface thereof. As illustrated, the aperture 37 includes a region of relatively larger diameter 38 coaxially aligned above a region of relatively smaller diameter 40. Both the relatively larger diameter and smaller diameter regions of the aperture are internally threaded as illustrated. In order to seal the aperture while still allowing ready access to the interior region of the housing, the upper housing member 30 also includes a removable cap 41 having a domed upper surface 42 and an externally threaded cylindrical lower portion 43 dimensioned as to engage the threads of the relatively larger diameter segment 38 of the aperature 37. To allow a tight seal between the cap and the housing, the upper housing member includes a raised annular seat 44 adjacent the periphery of the aperture against which the cap bears as it is screwed into the upper housing member.

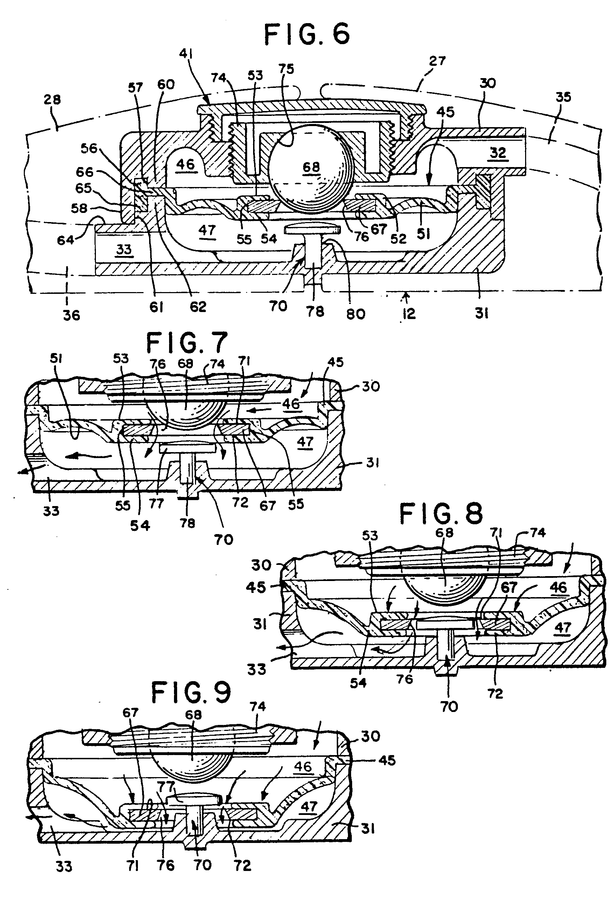

Referring further to figures 3 and 4, the valve includes partition means in the form of a movable member or diaphragm 45 extending laterally across the interior region of the inner housing thereby dividing that region into first and second interior chambers 46 and 47, respectively. The diaphragm 45 is fashioned from a flexible biocompatible material, such as silicone rubber, and, as best seen in fig. 3, comprises a disc-shaped member having an aperture 48 provided centrally therethrough. The operative surface 50 of the diaphragm is provided with an annular groove 51 concentrically aligned with the center aperture which allows the operative surface to travel vertically in response to differential pressure across the diaphragm such as might result from a difference in pressures in the first and second interior chambers. Toward its center, and in the region immediately surrounding the aperture, the thickness of the diaphragm is increased to form a raised area 52, while both the upper and lower surfaces of the raised area, 53 and 54 respectively, are undercut to form a circular, rectangular cross-sectioned channel 55 therebetween as is best seen in fig. 6. The diaphragm 45 also includes an integrally formed raised circular edge 56 projecting both above and below the operative surface 50 along its outer circumference. This edge, in a manner to be developed, facilitates installation of the diaphragm within the housing.

The manner in which the diaphragm is held in position relative to both the upper and lower housing members is best illustrated in figures 4 and 6. Referring to those figures, it will be seen that the lower edge of the upper housing member is provided with a rectangular cross section channel 57 thereby forming inner and outer sleeves 58 and 60 respectively. As illustrated, the vertical dimension of the inner sleeve 58 is less than that of the outer sleeve 60 while the channel 57 is dimensioned to receive the outer raised edge 56 of the diaphragm. The upper edge surface of the lower housing member is provided with a pair of raised steps 61 and 62 thereby forming concentric annular ledges 64, 65 and 66 thereon. When assembled, the lower edge of the outer sleeve 60 contacts the first ledge 64, while the second ledge 65 is dimensioned so as to contact the lower edge 56 of the diaphragm when the diaphragm is in place. Similarly, the inner ledge 64 is dimensioned as to allow the diaphragm to be received in the space formed between the upper edge thereof and the lower edge of the interior sleeve 60. Thus, when assembled, upper housing member 30 interlocks with lower housing member 31 by engagement of their corresponding edges, while the diaphragm 45 is received in the space provided therebetween thereby rigidly affixing the outer periphery of the diaphragm relative to the two interior housing members. When mounted in this manner, the operative surface 50 of the diaphragm is free to travel vertically in response to a pressure differential existing between fluids contained in the first and second chambers.

To regulate the passage of fluid from the first chamber 46 to the second chamber 47 and hence from a brain ventricle to the selected drainage area of the body, the valve includes valving means for regulating fluid communication between the first and second chambers. Such valving means take the form of a valve seat 67 mounted for movement between valve closure means and fluid restrictor means. In the embodiment illustrated, such valve closure means take the form of a sphere 68, fashioned from ruby, sapphire or similar hard, biocompatible material, while the fluid restrictor means take the form of a generally cylindrical restrictor 70 also fashioned from ruby, sapphire or similar such material. The valve seat 67, formed of the same material selected for the sphere and the restrictor, comprises a disc having flat, parallel, upper and lower faces 71 and 72. As is best seen in fig. 6, the valve seat is received within the channel 55 formed in the raised segment 52 of the diaphragm adjacent the aperture provided centrally therethrough whereby the valve seat travels with movement of the diaphragm. The ruby valve closure sphere 68 is positioned directly above the valve seat and is held in position by means of a cylindrical collar 74 externally threaded and dimensioned to engage the threads of the relatively narrow diameter segment 40 of the aperture provided in the upper surface of upper housing member 30 directly beneath the cap 41. The cylindrical collar 74 includes a central hollow region 75 dimensioned to receive the ruby sphere 68 and may be engagingly or disengagingly rotated relative to the upper housing member whereby the vertical position of the sphere relative to the valve seat 67 may be adjusted. As may further be seen in fig. 6, the valve seat 67 includes an orifice 76 extending centrally therethrough which provides fluid communication between the first and second chambers. As illustrated, the orifice 76 is located directly beneath the sphere 68 so that when the sphere contacts the upper surface of the valve seat 67, as would occur when the diaphragm 45 is deflected upwardly, it totally occludes the orifice thereby precluding the passage of fluid between the first and second chambers. The restrictor extends perpendicularly upward from the lower interior surface of the inner housing and is positioned directly beneath the valve seat orifice so that downward travel of the diaphragm and the valve seat results in the introduction of the restrictor 70 into the orifice thereby partially occluding the passage between the first and second chambers.

It will be observed that the orifice 76 provided in the valve seat is not a simple cylindrical aperture but rather is tapered so that the orifice 76 is narrowest at the upper surface 71 of the valve seat 67 and widest at its lower surface 72. The restrictor 70 is generally cylindrical in form and includes a segment of relatively larger diameter 77 above and coaxially aligned with a segment of relatively narrower diameter 78 the lower edge of which is received in a suitable recess 80 provided in the lower interior surface of housing member 31 thereby affixing the restrictor perpendicularly relative to that surface. The dimension of the restrictor is selected so that it will barely pass through the orifice at its narrowest point. By way of example, in one embodiment of the valve, the valve seat orifice had a diameter of .040 inches at its narrowest point and the clearance between the restrictor and the orifice at the narrowest point was on the order of 001 inches.

The operation of the valve may best be understood by reference to figures 6—10 and the description which follows. Fig. 10 is a graphical depiction of pressure versus flow characteristics of a valve constructed in accordance with the invention. Briefly, the valve operates to maintain a desired differential pressure $P_1$ between fluids in the brain ventricles and at the selected discharge location of the body. The valve accomplishes this by adjusting the fluid flow rate $Q$ so that the desired pressure $P_1$ is maintained. Such operation of the valve occurs within the region designated I on the graph of fig. 10.

When differential pressure rapidly increases, such as when the patient stands, a flow rate greater than a pre-selected rate $Q_1$ will be necessary to maintain the desired first pressure $P_1$. However, such a flow rate may create risks of undesirable hyperdrainage of the brain ventricles.

Accordingly, when such a rapid increase in differential pressure occurs, the valve automatically serves to maintain a relatively constant desired rate of fluid flow despite changes in differential pressure. In a practical valve, the flow rate will not be entirely independent of the applied differential pressure but rather will increase from a lower flow rate $Q_1$ to a higher flow rate $Q_2$ as differential pressure increases between first pressure $P_1$ and a second pressure $P_2$ as indicated by the solid line in fig. 10. A valve operating in this condition is operating in region II of fig. 10. Flow rates $Q_1$ and $Q_2$ are sufficiently low so that during a temporary rapid increase in differential pressure, pressure will return to normal before a quantity of CSF sufficient to cause adverse side effects may flow through the valve. In a typical valve $Q_1$ and $Q_2$ might be 0.4 ml./min and 0.8 ml./min. respectively, while first and second pressures, $P_1$ and $P_2$ may have values of 80 and 350 millimeters of water respectively. While it is desirable to avoid high flow rates through the valve in order to avoid hyperdrainage of the ventricles, it may, under certain emergency conditions, be desirable to allow rapid shunting of CSF in order to avoid possible brain damage. When the valve is operating in region II, increases in differential pressure tend to close the valve between the first and second interior chambers. To avoid the possibility of building extremely high ventricular CSF pressure, the valve is constructed so that when differential pressure exceeds a second pressure $P_2$ substantially higher than first pressure $P_1$, the valve once again operates to allow a fluid flow rate sufficient to maintain a differential pressure no higher than second pressure $P_2$. Such operation is that which would occur in region III of fig. 10. When the valve is operating in this region, further increases in differential pressure result in an increase in fluid flow through the valve thereby stabilizing differential pressure.

Figures 6—9 illustrate operation of the valve in each of the regions previously described. CSF applied to the inlet port 21 of the valve completely fills the first chamber 46 and exerts a downwardly directed force on the diaphragm 45 by reason of the CSF pressure within the brain ventricle. Since the second chamber 47 is in fluid communication with the selected drainage location in the body, the pressure of the CSF therein exerts an upwardly directed force on the lower surface of the diaphragm. Accordingly, the differential pressure between CSF in the brain ventricle and fluid at the drainage location results in verticle deflection of both the diaphragm and the valve seat 67 rigidly attached thereto. As shown in fig. 6, when valve seat 67 contacts sphere 68, the orifice 76 is totally occluded, thereby preventing fluid flow between chambers 46 and 47. When differential pressure is relatively low, such as when the valve is operating in region I fig. 10, the resulting slight downward displacement of the diaphragm is sufficient to displace the valve seat 67 relative to the sphere 68 thereby allowing fluid to pass through the orifice 76 from the first interior chamber 46 to the second interior chamber 47. The valving means in this condition are illustrated in fig. 7. As shown, downward deflection of the diaphragm is sufficient to allow the passage of fluid through the orifice, yet the upper surface of the restrictor 70 is sufficiently removed from the orifice so as not to interfere with the flow of fluid between the chambers. In this condition, the valve acts primarily as a constant pressure device whereby a constant pressure differential is maintained between the fluids in the first and second chambers. A slight increase in differential pressure results in slight downward deflection of the diaphragm thereby further opening the valving means to allowgreater fluid flow between the chambers with the results that the increased pressure in the first chamber is rapidly relieved. Similarly, a decrease in the pressure of fluid in the first chamber allows the diaphragm to move toward the sphere, thereby restricting the fluid flow between the chambers, thus allowing pressure in the first chamber to increase.

Fig. 8 illustrates the condition of the valve when a sudden increase in differential pressure is applied to the valve. When such an event occurs, the pressure differential exceeds the first desired pressure $P_1$ and consequently the valve operates in region II of fig. 10. When such a differential pressure is applied to the valve, the downward displacement of the diaphragm 45 is sufficient to cause the valve seat 67 to descend over the restrictor 70 thereby causing the restrictor to partially occlude the orifice 76 therein. Because the orifice is tapered, additional downward travel of the valve seat results in a further occlusion of the orifice. The orifice is shaped such that the additional occlusion occurring orifice by reason of increasing differential pressure is sufficient to offset the higher flow rate ordinarily resulting from increased pressure thereby resulting in a relatively uniform rate fluid flow between the chambers despite increase in differential pressure. Accordingly, in this condition, the valve acts primarily as a constant flow device permitting the passage of fluid from the first to the second chamber at a relatively constant desired rate despite changes in applied differential pressure.

Fig. 9 illustrates operation of the valve in region III of fig. 10 such as would occur when differential pressure exceeds the desired second pressure $P_2$.

In this condition, differential pressure displaces the diaphragm to a degree sufficient to cause the large diameter end 77 of the restrictor 70 to protrude past the upper surface of the valve seat thereby allowing fluid to easily flow past the restrictor and through the orifice 76. It will be noted, that in this condition, the orifice is not nearly as restricted as it was in fig. 8 when the large diameter end of the restrictor 70 was received within the tapered egion of the valve seat 67. When the valve is operating in this manner, increases in differential pressure cause the valve seat to be further displaced away from the restrictor, thereby further opening the orifice, and allowing a greater fluid flow rate. Thus, the valve once again operates essentially as a constant pressure device whereby differential pressure greater than a desired maximum pressure $P_2$ is prevented. A further advantage of this construction, is that as the restrictor passes through the orifice, it tends to remove foreign materials which may tend to clog the valve. Thus, should clogging occur, the resulting increased differential pressure will eventually cause the restrictor to pass through the orifice thereby providing the valve with a self-cleaning feature.

**Claims**

1. A valve for controlling the passage of body fluids (14) from a source location in the body, wherein the fluid (14) is normally subject to pressure variations within a predetermined nominal range, and abnormally subject to pressure variations in excess thereof extending to a predetermined maximum allowable level, to a drain location in the body which is at relatively constant pressure, comprising:

a housing (26) having first (46) and second (47) interior chambers;

inlet port means for establishing fluid communication between said first chamber (46) and the source location;

outlet port means for establishing fluid communication between said second chamber (46) and the drain location.

valving means for regulating fluid flow between said first chamber (46) and said second chamber (47), said valving means providing a first condition in which fluid communication between said first (46) and second (47) chambers is prevented and a second condition in which fluid flow between said first (46) and second (47) chambers is established; and

partition means in said housing including a movable member (45) separating said first (46) and second (47) chambers and being movable in response to the pressure differential therebetween, said movable member (45) being operatively associated with said valving means such that motion of said movable member (45) in response to an increasing pressure differential between fluid in said first (46) and second (47) chambers conditions said valving means from said first condition into said second condition, characterized in that said valving means in said second condition regulates the fluid flow between said first (46) and said second (47) chambers to maintain a first substantially constant predetermined pressure in said first chamber (46);

said valving means providing a third condition in which fluid flow between said first (46) and second (47) chambers is maintained at a substantially constant flow rate, and a fourth condition in which fluid flow between said first (46) and second (47) chambers is regulated to maintain a second substantially constant predetermined pressure in said first chamber (46), wherein said movable member (45) sequentially moves from said first condition through said second and third conditions to said fourth condition whereby, in response to an increasing pressure differential between fluid at the source location and fluid at the drain location, said valving means in said first condition prevents the passage of fluid between the source location and the drain location when the source pressure falls below the nominal range, said valving means in said second condition maintains a constant fluid pressure differential between the source location and the drain location when the source pressure is in the nominal range, said valving means in said third condition maintains a desired constant rate of fluid flow between the source location and the drain location when the source pressure is greater than the nominal range but less than the maximum allowable level, and said valving means in said fourth condition maintains said maximum allowable pressure level at the source location.

2. A valve as defined in claim 1 wherein said valving means comprises:

valve closure means (68) mounted within said housing (26);

restrictor means (70) mounted in said housing (26) opposite said valve closure means (68) and spaced apart therefrom; and valve seat means (67) having an orifice (76) therethrough, said orifice (76) being dimensioned as to allow the passage of said restrictor means (70) therethrough, said valve seat means (67) being disposed between said valve closure means (68) and said restrictor means (70) and mounted for movement from a first position in which said valve seat means (67) move into closing relationship with said valve closure means (68) thereby closing said valving means, to a second position in which said restrictor means (70) extend through said orifice (76) of said valving means to partially restrict the movement of fluid therethrough.

3. A valve as defined in claim 1 or 2 wherein said movable member (45) comprises a diaphragm (45) which defines said first (46) and second (47) chambers.

4. A valve as defined in claim 2 or 3 wherein said valve seat means (67) are carried on said diaphragm (45).

5. A valve as defined in one of the claims 2 to 4 wherein said valve closure means (68) comprise a sphere (68).

6. A valve as defined in one of the claims 2 to 5 wherein said restrictor (70) comprises a generally cylindrical member attached at one end to an interior surface of said housing (26).

7. A valve as defined in claim 6 wherein said orifice (76) is tapered such that the narrowest region of said orifice (76) is nearest said valve closure member (68) and the widest region of said orifice (76) is nearest said cylindrical member.

8. A valve as defined in one of the claims 2 to 7 wherein said housing (26), said valve seat (67), said valve closure means (68) and said fluid flow restrictor means (70) are of bio-compatible material.

**Patentansprüche**

1. Ein Ventil zur Steuerung des Durchganges von Körperflüssigkeiten (14) von einer Quellstelle im Körper, worin die Flüssigkeit (14) normalerweise innerhalb eines vorbestimmten Sollbereiches Druckschwankungen ausgesetzt ist und außerhalb dieses anormalerweise übermäßigen Druckschwankungen ausgesetzt ist, die sich auf ein vorbestimmtes maximal zu erlaubendes Niveau ausdehnen, bis zu einer Drainstelle im Körper, die einen relativ konstanten Druck aufweist mit:

einem Gehäuse (26) mit erster (46) und zweiter (47) innerer Kammer;

Einlaßkanalmittel zum Errichten einer Flüssigkeitsverbindung zwischen der ersten Kammer (46) und der Quellstelle;

Auslaßkanalmittel zur Errichtung einer Flüssigkeitsverbindung zwischen der zweiten Kammer (46) und der Drainstelle;

Ventilmittel zum Regulieren des Flüssigkeitsflusses zwischen der ersten Kammer (46) und der zweiten Kammer (47), wobei besagtes Ventilmittel einen ersten Zustand schafft, in welchem Flüssigkeitsverbindung zwischen der ersten (46) und zweiten (47) Kammer verhindert wird und einen zweiten Zustand, in welchem Flüssigkeitsfluß zwischen der ersten (46) und zweiten (47) Kammer hergestellt ist; und Trennmittel in dem Gehäuse, ein bewegliches Bauteil (45) einschließend, welches die erste (46) und die zweite (47) Kammer voneinander trennt und welches in Reaktion auf den Druckunterschied dazwischen beweglich ist, wobei das bewegliche Bauteil (45) dem Ventilmittel betriebsmäßig so zugeordnet ist, daß die Bewegung des beweglichen Bauteiles (45) als Antwort auf einen anwachsenden Unterschiedsdruck zwischen Flüssigkeit in der ersten (46) und zweiten (47) Kammer das Ventilmittel aus dem ersten Zustand in den zweiten Zustand bringt, dadurch gekennzeichnet, daß das Ventilmittel in dem zweiten Zustand den Flüssigkeitsfluß zwischen der ersten (46) und der zweiten (47) Kammer reguliert, um einen ersten im wesentlichen konstanten vorbestimmten Druck in der ersten Kammer (46) aufrechtzuerhalten;

das Ventilmittel einen dritten Zustand zu Verfügung stellt, in welchem der Flüssigkeitsfluß zwischen der ersten (46) und zweiten (47) Kammer mit einer im wesentlichen konstanten Flußrate aufrecht erhalten wird und einen vierten Zustand, in welchem der Flüssigkeitsfluß zwischen der ersten (46) und zweiten (47) Kammer reguliert wird, um einen zweiten im wesentlichen konstanten vorbestimmten Druck in der ersten Kammer (46) aufrechtzuerhalten, wobei das bewegliche Bauteil (45) sich nacheinander von dem ersten Zustand durch die zweiten und dritten Zustände zu dem vierten Zustand bewegt, wodurch als Antwort auf einen anwachsenden Unterschiedsdruck zwischen Flüssigkeit an der Quellstelle und Flüssigkeit an der Drainstelle das Ventilmittel im ersten Zustand den Flüssigkeitsübertritt zwischen der Quellstelle und der Drainstelle verhindert, wenn der Quelldruck unterhalb den Sollbereich fällt, wobei das Ventilmittel im zweiten Zustand einen konstanten Flüssigkeitsdruckunterschied zwischen der Quellstelle und der Drainstelle aufrecht erhält, wenn der Quelldruck innerhalb des Sollbereiches liegt, wobei das Ventilmittel im dritten Zustand eine gewünschte konstante Rate eines Flüssigkeitsflusses zwischen der Quellstelle und der Drainstelle aufrecht erhält, wenn der Quelldruck größer als der Sollbereich ist, jedoch kleiner als das maximal zulässige Niveau und wobei die Ventileinrichtung im vierten Zustand das maximal zulässige Druckniveau der Quellstelle aufrechterhält.

2. Ein Ventil nach Anspruch 1, in welchem das Ventilmittel umfaßt:

Ventilschlußmittel (68), das innerhalb des Gehäuses (26) montiert ist;

Durchflußbegrenzermittel (70), das in dem Gehäuse (26) gegenüber dem Ventilschlußmittel (68) montiert ist und räumlich getrennt davon angeordnet ist; und

Ventilsitzmittel (67) mit einer Öffnung (76) hindurch, wobei die Öffnung (76) so bemessen ist, daß sie den Durchtritt des Durchflußbegrenzermittels (70) dadurch erlaubt, wobei das Ventilsitzmittel (67) angeordnet ist zwischen dem Ventilschlußmittel (68) und dem Durchflußbegrenzermittel (70) und montiert ist zur Bewegung aus einer ersten Stellung, in welcher das Ventilsitzmittel (67) sich in schließender Beziehung mit dem Ventilschlußmittel (68) bewegt und dabei das Ventilmittel schließt, in eine zweite Stellung, in welcher das Durchflußbegrenzungsmittel (70) sich durch die Öffnung (76) des Ventilmittels erstreckt, um teilweise die Bewegung der Flüssigkeit dadurch zu begrenzen.

3. Ein Ventil nach Anspruch 1 oder 2, wobei das bewegliche Bauteil (45) ein Diaphragma (45) umfaßt, welches die erste (46) und zweite (47) Kammer definiert.

4. Ein Ventil nach Anspruch 2 oder 3, worin das Ventilsitzmittel (67) auf dem Diaphragma (45) getragen wird.

5. Ein Ventil nach einem der Ansprüche 2 bis 4, worin das Ventilschlußmittel (68) eine Kugel (68) umfaßt.

6. Ein Ventil nach einem der Ansprüche 2 bis 5, worin der Durchflußbegrenzer (70) ein im allgemeinen zylindrisches Bauteil umfaßt, welches an einem Ende an der inneren Oberfläche des Gehäuses (26) angebracht ist.

7. Ein Ventil nach Anspruch 6, worin sich die Öffnung (76) derart verjüngt, daß der schmalste Bereich der Öffnung (76) dem Ventilschlußbauteil (68) am nächsten gelegen ist und der weiteste Bereich der Öffnung (76) dem zylindrischen Bauteil am nächsten gelegen ist.

8. Ein Ventil nach einem der Ansprüche 2 bis 7, worin das Gehäuse (26), der Ventilsitz (67), das Ventilschlußmittel (68) und das Flüssigkeitsdurchflußbegrenzermittel (70) aus biokompatiblem Material sind.

## Revendications

1. Une valve pour commander le passage de fluides corporels (14) d'un emplacement de source dans le corps, dans lequel le fluide (14) est normalement soumis à des variations de pression dans une plage nominale prédéterminée, et est soumis anormalement à des variations de pression qui dépassent cette plage et s'élèvent jusqu'à un niveau maximal admissible prédéterminé, vers un emplacement d'évacuation dans le corps qui est à une pression relativement constante, comprenant:

un boîtier (26) ayant des première (46) et seconde (47) chambres intérieures;

des moyens de passage d'entrée pour établir une communication pour le fluide entre la première chambre (46) et l'emplacement de source;

des moyens de passage de sortie pour établir une communication pour le fluide entre la seconde chambre (46) et l'emplacement d'évacuation;

une structure de valve pour réguler le passage du fluide entre la première chambre (46) et la seconde chambre (47), cette structure de valve procurant une première condition dans laquelle il n'y a pas de communication pour le fluide entre la première chambre (46) et la seconde chambre (47), et une seconde condition dans laquelle une circulation de fluide entre la première chambre (46) et la seconde chambre (47) est établie; et

des moyens de séparation incorporés dans le boîtier, comprenant une pièce mobile (45) qui sépare la première chambre (46) et la seconde chambre (47) et qui se déplace sous l'effet de la différence de pression entre elles, cette pièce mobile (45) étant fonctionnellement assosiée à la structure de valve de façon que le mouvement de la pièce mobile (45) sous l'effet d'une différence de pression croissante entre le fluide dans la première chambre (46) et dans la seconde chambre (47) fasse passer la structure de valve de la première condition à la seconde condition, caractérisée en ce que la structure de valve dans la seconde condition régule la circulation du fluide entre la première chambre (46) et la seconde chambre (47), pour maintenir une pression prédéterminée pratiquement constante dans la première chambre (46);

la structure de valve procure une troisième condition dans laquelle la circulation du fluide entre la première chambre (46) et la seconde chambre (47) est maintenue à un débit pratiquement constant, et une quatrième condition dans laquelle la circulation du fluide entre la première chambre (46) et la seconde chambre (47) est régulée pour maintenir une seconde pression prédéterminée pratiquement constante dans la première chambre (46),

la pièce mobile (45) se déplace séquentiellement de la position correspondant à la première condition à la position correspondant à la quatrième condition, en passant par les positions correspondant aux seconde et troisième conditions, grâce à quoi sous l'effet d'une différence de pression croissante entre le fluide à l'emplacement de source et le fluide à l'emplacement d'évacuation, la structure de valve dans la première condition empêche le passage du fluide entre l'emplacement de source et l'emplacement d'évacuation lorsque la pression de la source tombe au-dessous de la plage nominale, la structure de valve dans la seconde condition maintient une différence de pression de fluide constante entre l'emplacement de source et l'emplacement d'évacuation lorsque la pression de la source est dans la plage nominale, la structure de valve dans la troisième condition maintient un débit de fluide constant désiré entre l'emplacement de source et l'emplacement d'évacuation lorsque la pression dela source est supérieure à la plage nominale mais inférieure au niveau maximal admissible, et la structure de valve dans la quatrième condition maintient le niveau de pression maximale admissible à l'emplacement de source.

2. Une valve selon la revendication 1, dans laquelle la structure de valve comprend:

des moyens de fermeture de valve (68) montés à l'intérieur du boîtier (26).

des moyens de restriction (70) montés dans le boîtier (26) face. aux moyens de fermeture de valve (68) et dans une position espacée par rapport à ces derniers; et un siège de valve (67) traversé par un orifice (76), cet orifice (76) étant dimensionné de façon que les moyens de restriction (70) puissent le traverser, le siège de valve (67) étant disposé entre les moyens de fermeture de valve (68) et les moyens de restriction (70), et étant monté de façon à effectuer un mouvement d'une première position dans laquelle le siège de valve (67) se positionne dans une relation de fermeture par rapport aux moyens de fermeture de valve (68), ce qui a pour effet de fermer la structure de valve, vers une seconde position dans laquelle les moyens de restriction (70) s'étendent à travers l'orifice (76) de la structure de valve, pour restreindre partiellement le passage du fluide à travers cet orifice.

3. Une valve selon la revendication 1 ou 2, dans laquelle la pièce mobile (45) consiste en un diaphragme (45) qui définit la première chambre (46) et la seconde chambre (47).

4. Une valve selon la revendication 2 ou 3, dans laquelle le siège de valve (67) est supporté par le diaphragme (45).

5. Une valve selon l'une des revendications 2 à 4, dans laquelle les moyens de fermeture de valve (68) consistent en une sphère (68).

6. Une valve selon l'une des revendications 2 à 5, dans laquelle les moyens de restriction (70) consistent en une pièce de forme générale cylindrique dont une extrémité est fixée à une surface intérieure du boîtier (26).

7. Une valve selon la revendication 6, dans laquelle l'orifice (76) est tronconique, de façon que la région la plus étroite de cet orifice (76) soit la plus proche des moyens de fermeture de valve (68), et que la partie la plus large de l'orifice (76) soit la plus proche de la pièce cylindrique.

8. Une valve selon l'une des revendications 2 à 7, dans laquelle le boîtier (26), le siège de valve (67), les moyens de fermeture de valve (68) et les moyens de restriction de circulation de fluide (70) sont constitués par des matériaux biocompatibles.

FIG. 1

FIG. 2

FIG. 3

# FIG. 4

# FIG. 5

# FIG. 10

EP 0 156 974 B1

FIG. 6

FIG. 7

FIG. 8

FIG. 9

3